(19) Europäisches Patentamt
European Patent Office
Office européen des brevets

(11) EP 2 653 159 B1

(12) **EUROPEAN PATENT SPECIFICATION**

(45) Date of publication and mention of the grant of the patent:
**29.06.2016 Bulletin 2016/26**

(21) Application number: **11848793.3**

(22) Date of filing: **13.12.2011**

(51) Int Cl.:
***A61K 31/196*** *(2006.01)* ***A61P 29/00*** *(2006.01)*
***A61P 27/02*** *(2006.01)* ***A61K 31/13*** *(2006.01)*
***A61K 31/131*** *(2006.01)* ***C07C 213/08*** *(2006.01)*
***C07C 215/40*** *(2006.01)* ***C07C 227/14*** *(2006.01)*
***C07C 229/40*** *(2006.01)*

(86) International application number:
**PCT/CN2011/083873**

(87) International publication number:
**WO 2012/079497 (21.06.2012 Gazette 2012/25)**

(54) **BROMFENAC ORGANIC SALTS AND PREPARATION METHOD, COMPOSITION AND USE THEREOF**

ORGANISCHE BROMFENACSALZE UND HERSTELLUNGSVERFAHREN DAFÜR, ZUSAMMENSETZUNG DARAUS UND IHRE VERWENDUNG

SELS ORGANIQUES DE BROMFÉNAC ET PROCÉDÉ DE PRÉPARATION, COMPOSITION ET UTILISATION DE CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2010 CN 201010612766**

(43) Date of publication of application:
**23.10.2013 Bulletin 2013/43**

(73) Proprietor: **Shenyang Xingqi Pharmaceutical Co., Ltd.**
**Liaoning 110027 (CN)**

(72) Inventors:
- **LIU, Jidong**
  **Shenyang, Liaoning 110027 (CN)**
- **YANG, Yuchun**
  **Shenyang, Liaoning 110027 (CN)**
- **WANG, Jiuliang**
  **Shenyang, Liaoning 110027 (CN)**
- **TANG, Hai**
  **Shenyang, Liaoning 110027 (CN)**

(74) Representative: **Inspicos P/S**
**Kogle Allé 2**
**2970 Hørsholm (DK)**

(56) References cited:
**WO-A1-2008/017903** **CN-A- 101 313 899**
**US-A- 5 554 789** **US-A1- 2003 055 051**
**US-A1- 2007 021 507** **US-A1- 2007 082 857**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 653 159 B1

## Description

### Technical Field

The present invention relates to pharmacology and pharmaceutical pharmacy, and relates to a bromfenac organic salt. The present invention further relates to a preparation method of the bromfenac organic salt, a pharmaceutical composition comprising the bromfenac organic salt, the bromfenac organic salt or the composition comprising the same for use as a medicament, e.g. for the treatment and/or prophylaxis of inflammation or as a medicament for analgesia.

### Background Art

Bromfenac sodium is a non-steroid anti-inflammatory agent developed by Wyeth-Ayerst Lab Inc, and is a derivative of 2-amino-3-benzoyl-phenylacetic acid (Amfenac), which went on sale in the United States in 1997; bromfenac sodium has potent analgesic effect, and is used for treatment of sharp pain while having no addiction, and its potency is 10 times of that of other non-steroid anti-inflammatory agents. Bromfenac sodium is the most effective cyclooxygenase inhibitor, which results in anti-inflammatory, anti-anaphylaxis and analgesia effects mainly by inhibiting cyclooxygenase to block synthesis of prostaglandins inflammation regulators.

Bromfenac sodium has a chemical name of 2-amino-3-(4-bromobenzoyl) phenylacetic acid sodium salt sesquialter hydrate, and has a chemical structure as follows:

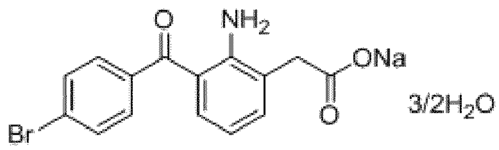

Bromfenac sodium

Bromfenac sodium is soluble in water, almost insoluble in 0.1 M hydrochloric acid solution, acetonitrile and acetone, soluble in methanol, and slightly soluble in ethanol.

Bromfenac sodium ophthalmic solution was developed by Senju Company of Japan, and went on sale in Japan in 2000, which is a 5mL/bottle 0.1% Bromfenac sodium ophthalmic solution, and used for treatment of inflammatory diseases at external ocular regions and front ocular regions, such as blepharitis, conjunctivitis, keratohelcosis and postoperative inflammations.

US 2007/021507 A1 discloses agueous eye drops with accelerated intraoccular migration. The eye drops contain bromfrenac and an organic amine, in particulcar aminoethylsulfonyl acid or trometamol.

US 2007/082857 A1 discloses aqueous preparations containing an aminoglycoside antibiotic and bromfenac. The preparation may contain an organic base.

US 2003/055051 A1 discloses an ophthalmic aqueous preparation comprising meloxicam and trometamol.

### Contents of the Invention

After inventive works and unremitting efforts, the inventors of the present invention found a new Bromfenac organic salt, which has good anti-inflammatory and analgesia activity and has good stability, and at the meantime, the inventors also surprisedly found that it has transdermic properties and tissue distribution concentrations superior to those of bromfenac sodium. Thus, the present invention is provided as follows:

One aspect of the present invention relates to a bromfenac organic salt, which is bromfenac diethylamine salt, cf. Formula II, or bromfenac trometamol salt, cf. Formula III:

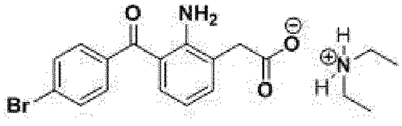

II

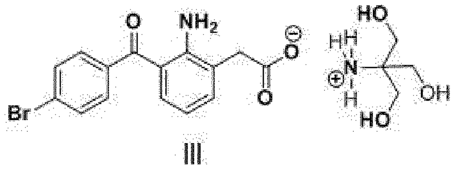

III

Another aspect of the present invention relates to a method for the synthesis of the bromfenac organic salt of

the present invention, comprising the following steps:

1) dissolving bromfenac in an organic solvent, heating to dissolve, then adding an organic base in equimolar amount, stirring until the end of reaction (using thin-layer chromatography to determine the end of reaction);
2) concentrating and evaporating the reaction solution to dry to obtain a light yellow or orange yellow solid;
3) recrystallizing the solid obtained in step 2) to obtain the bromfenac organic salt;

wherein the said organic base is diethylamine or trometamol.

In one embodiment of the present invention, said organic solvent in step 1) is one or more selected from the group consisting of methanol, ethanol, acetone, n-butanol, and toluene, specifically, is acetone; said organic base is a nitrogen-containing organic base, specifically, is diethylamine or trometamol.

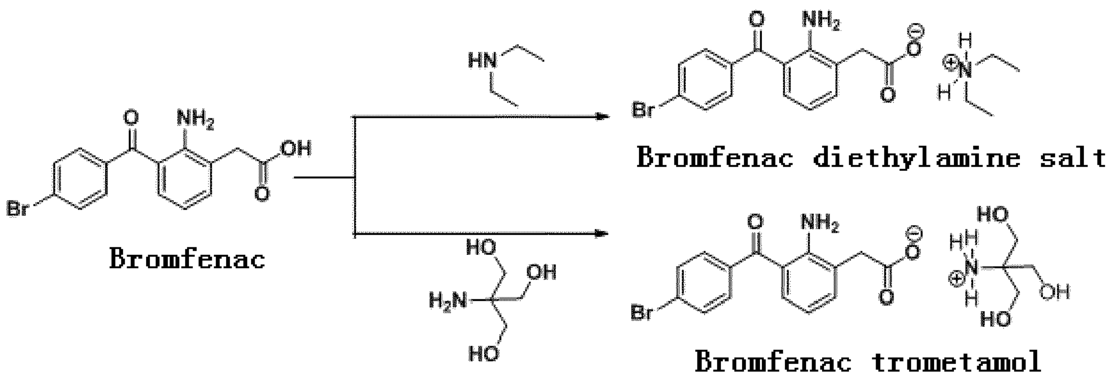


In one embodiment of the present invention, the temperature of heating in step 1) is 25-80°C; specifically, is 45-60°C, more specifically, is 55°C.

In one embodiment of the present invention, the stirring reaction in step 1) is performed for a time of 0.5-2 hours; specifically, is 1 hour.

In one embodiment of the present invention, the recrystallizing in step 3) is performed at 0°C or below; specifically, at -5°C.

In one embodiment of the present invention, the preparation method further comprises the following steps:

4) vacuum drying the crystal obtained by recrystallizing.

Further another aspect of the present invention relates to a pharmaceutcial composition, which comprises the bromfenac organic salt of the present invention, wherein the composition is in a dosage form selected from a tablet, a capsule, a dripping pill, an aerosol, a pills, a powder, an emulsion, a granule, a liposome, a transdermal agent, a buccal tablet, a suppository, and a freeze-dried powder injection. Optionally, the composition further comprises a pharmaceutically acceptable carrier or excipient.

In the present invention, the term "composition" refers to a pharmaceutical composition, which can be applied to a subject to achieve treatment, prophylaxis, alleviation and/or relief of the diseases or disorders of the present invention. In one embodiment of the present invention, the pharmaceutical composition is a medicament for treatment and/or prophylaxis of inflammations or a pharmaceutical composition for analgesia.

Usually, the pharmaceutical composition of the present invention contains 0.01-90wt% of the compound of the present invention. The pharmaceutical composition can be prepared according to methods known in the art, and can be processed into a suitable administration form or dosage form for human application.

The compound or the pharmaceutical composition containing the compound can be administered in a unit dosage form, and administration routes can be intestinal route or parenteral route, such as oral, intramuscular, subcutaneous, intranasal, buccal, percataneous, peritoneal, rectal routes. The dosage form can be tablets, capsules, dripping pills, aerosols, pills, powders, solutions, suspensions, emulsions, granules, liposomes, transdermal agents, buccal tablets, suppositories, and freeze-dried powder injections. It can be normal preparations, or sustained release preparations, controlled release preparations as well as various particulate delivery systems. In order to obtain tablets as unit dosage form, various carriers known in the art can be used. The examples of carriers are, such as, diluents and absorbers, such as starch, dextrin, calcium sulfate, lactose, mannitol, sucrose, sodium chloride, glucose, urea, calcium carbonate, kaolin, microcrystalline cellulose, aluminum silicate; wetting agents and binders, such as water, glycerol, polyethylene glycol, ethanol, propanol, starch paste, dextrin, syrup, honey, glucose solution, acacia mucilage, gelatin paste, sodium carboxymethylcellulose, shellac, methylcellulose, potassium phosphate, polyvinylpyrrolidone; disintegrants, such as dry starch, alginate, agar powder, laminarin powder, sodium hydrogen carbonate and citric acid, calcium carbonate, polyoxyethylene, sorbitol fatty acid esters, sodium dodecyl sulfate, methylcellulose, ethylcellulose; disintegration inhibitors, such as sucrose, tristearin, cacao butter, hydrogenated oil; absorption enhancers, such as quaternary ammonium salt, sodium dodecyl sulfate; lubricants, such as talc powder, silica, corn starch, stearates, boric acid, liquid paraffin, polyethylene glycol. The tablets can be further processed to form coated tablets, such as sugar coated tablets, thin membrane coated tablets, enteric-coated tablets, or double layer tablets or multiple layer tablets. In order to obtain pills as unit dosage form, various carriers known in the art can be used. The examples of these carriers can be, such as diluents and absorbers, such as glucose, lactose, starch, cacao butter, hydrogenated oil, polyvinylpyrrolidone, Gelucire, kaolin, talc powder; binders such as acacia gum, tragacanth gum, gelatin, ethanol, honey, liquid sugar, rice paste or panada; disintegrants, such as agar powder, dry starch, alginates, sodium dodecyl sulfate, methylcellulose, ethylcellulose. In order to obtain suppositories as unit dosage form, various carriers known in the art can be used. The examples of these carriers are, such as polyethylene glycol, lecithin, cacao butter, fatty alcohols, esters of fatty alcohols, gelatin, and semi-synthesized glycerides. In order to obtain capsules as unit dosage form, the compound of Formula I or a stereoisomer thereof as effective component is blended with various carriers, and the resultant mixture is placed in a hard gelatin capsule or a soft capsule. The compound of Formula I or a stereoisomer thereof as effective component can also be processed to obtain microcapsules, suspended in aqueous medium to obtain a suspension, or loaded in hard capsules or processed to obtain injections. In order to obtain injections as unit dosage form, such as solutions, emulsions, freeze-dried injection powders or suspensions, all diluents commonly used in the art can be used, such as, water, ethanol, polyethylene glycol, 1,3-propanediol, ethoxylated isostearyl alcohol, multiple-oxidized isostearyl alcohol, polyoxyethylene sorbitan fatty acid ester. In addition, in order to obtain iso-osmia injections, a suitable amount of sodium chloride, glucose or glycerol can be added to injection preparation. Furthermore, conventional co-solvents, buffering agents, pH regulators and so on can also be added.

In addition, if desired, coloring agents, preservatives, perfumes, correctants, sweet tastes or other materials can also be added to the pharmaceutical preparations.

The actual dose levels of various active components in the pharmaceutical composition of the present invention can be changed so that the active compounds are in effective levels for specific patients, compositions and administration routes, and desired therapeutic effects can be achieved. Dosage level can be determined according to activity of specific compound, administration route, severity of disease to be treated, and conditions and medical history of patient. However, a method in the art comprises starting from a dose of compound that is lower than the level as required for achieving desired therapeutical effects, gradually increasing the dose until desired effects are achieved.

Further another aspect of the present invention relates to the Bromfenac organic salt or the pharmaceutical composition of the present invention for use as a medicament, such as for the treatment and/or prophylaxis of inflammation or as a medicament for analgesia.

The inflammation comprises inflammations of various tissues of human body (such as skin inflammations, aural inflammations such as auricle inflammations or auricle swelling, foot swelling); specifically, the inflammation is an inflammation associated with or mediated by prostaglandin, more specifically, is ocular inflammations such as blepharitis, conjunctivitis, keratohelcosis, or ocular postoperative inflammations. The analgesia comprises relieving pains of various sites of human body; specifically, which are pains caused or mediated by prostaglandin; more specifically, are pains caused by ocular diseases.

Prostaglandin can enhance ability of other inflammatory mediators in inducing inflammation, promoting development of inflammation, and at the same time has hyperalgesia effect which increase sensitivity of pain receptors for pain-producing substances such as bradykinin, and prostaglandin itself also has pain-producing effect.

The action mechanism of non-steroid anti-inflammatory agents is to inhibit cyclooxygenase, block biosynthesis and release of prostaglandin, prevent eye from stimulation and injury caused by inflammation mediators, so that it has good potency of antianaphylaxis, relieving pruritus, antiphlogosis and analgesia. Hence, non-steroid anti-inflammatory agents inhibiting prostaglandin synthesis have effects of antiphlogosis as well as analgesia. In addition, non-steroid anti-inflammatory agents have effects of inhibiting myosis induced by operation, and can maintain mydriasis during operation; so that non-inflammatory agents can be used in advance or at early stage after injury and exhibit significant inhibition effects on inflammatory reaction and complications.

The dosage of administration of the compound (bromfenac organic salt) or composition of the present invention depends on many factors, such as properties and severity of the disease to be prevented or treated, gender, age, bodyweight and individual reaction of the patient or animal, the specific compound to be used, administration route and times of administration, etc. The above dosage can be single dosage or constitutes of several sub-dosages, such as two, three or four sub-dosages for administration.

In the present invention, the term "effective amount" refers to a dose that can achieve treatment, prophylaxis, alleviation and/or relief of the diseases or disorders of the present invention.

The term "subject" refers to a patient or an animal which is subjected to the composition of the present invention

for treatment, prophylaxis, alleviation and/or relief of the disease or disorder of the present invention, especially a mammal, such as human, dog, monkey, cattle or horse.

The term "disease and/or disorder" refers to a physical state of the subject, and the physical state relates to the disease and/or disorder of the present invention.

Beneficial Effects of the Invention

The present invention provides a new bromfenac organic salt, which has effects of anti-inflammation and analgesia, good water solubility and lipo-solubility, good stability after long-term storage. In addition, in comparison with bromfenac sodium in the market, the compound of the present invention has better transdermal effects and tissue distribution concentration.

The preparation method of the present invention is of simple process, readily available raw materials, stable reaction conditions, high reaction yield, high purity of product, and has less pollutions of three-wastes.

## Specific Model for Carrying Out the Invention

The embodiments of the present invention are illustrated in details in conjunction with the following examples. However, those skilled in the art would understand that the following examples are merely used to illustrate the present invention.

Specific means or conditions that are not given in the examples were conducted according to the documents in the art or specifications. The reagents or instruments which manufacturers are not given are all conventional products commercially available in the market.

Example 1: Preparation of bromfenac diethylamine salt

3.8g (0.0113mol) of bromfenac was precisely weighed and placed in a dry and clean 100mL three-necked bottle, added with 60mL of acetone, stirred, heated at 55°C until dissolved, added to the reactor with 1.2mL (0.0113mol) of diethylamine, continuously heated until precipitate dissolved completely, stirred and reacted at 55°C for 1 hours, until the end of reaction (the end of reaction was determined by thin-layer chromatography), the solvent was removed by reduced pressure distillation to obtain a light yellow solid, to which 28 mL of a mixture solvent of acetone and water (volume ratio was 3:1) was added, refluxed until the light yellow solid dissolved completely, a small amount of active carbon was added and further stirred for 10 min, filtered when warm, the filtrate was placed in a refrigerator overnight (cooled and crystallized at -5°C), filtered, the filter cake was vacuum dried to obtain 2.7 g of a light yellow crystal. mp: 151-153°C, yield 58.7%.

The product was subjected to element analysis, measured values (theoretical values): C 56.03(56.07), H 5.69(5.46), Br 19.62(19.67), N 6.88(6.89), O 11.78(11.81), $^1$HNMR(300 MHz, $d_6$-DMSO): δ1.67(t, 6H, $-CH_3$) δ3.29 (s, 2H, ArCH2 $COO^-$), δ3.37(q, 4H, $-CH_2$-N-), δ6.47 (t, 1 H, *J* = 7.5Hz, N-Ar$^5$H), δ7.10 (d, 1H, *J* = 7.8Hz, N-ArH), δ7.17 (d, 1H, *J* =6.8Hz, N-ArH), δ7.49 (d, 2H, *J* = 8.3Hz, Br-ArH), δ7.71 (d, 2H, *J* = 8.3Hz, Br-ArH), δ7.01 (s, 2H, $NH_2^+$), δ7.88 (bro, 2H, Ar-$NH_2$). The analysis results of MS, UV, IR, and HPLC showed the product was bromfenac diethylamine salt, and had a purity of 99.5%.

Example 2: Preparation of bromfenac trometamol salt

3.8g (0.0113mol) of bromfenac was precisely weighed and placed in a dry and clean 100mL three-necked bottle, added with 80mL of acetone, stirred, heated to 55°C until dissolved, added to the reactor with 1.4g (0.0113mol) of trometamol, continuously heated until precipitate dissolved completely, stirred at 55°C for 1 until the end of reaction (the end of reaction was determined by thin-layer chromatography), the solvent was removed by reduced pressure distillation to obtain a orange yellow solid, to which 33 mL of a mixture solvent of ethanol and water (volume ratio was 9:1) was added, refluxed until the orange yellow solid dissolved, a small amount of active carbon was added and continuously stirred for 10 min, filtered when warm, the filtrate was placed in a refrigerator overnight (cooled and crystallized at -5°C), filtered, the filter cake was vacuum dried to obtain 3.5 g of a light yellow crystal. Mp: 144-147°C, yield 67.3%.

The product was subjected to element analysis, measured values (theoretical values): C 50.12(50.15), H 5.09(5.08), Br 17.55(17.52), N 6.15(6.15), O 21.05(21.08). $^1$HNMR(300 MHz,$d_6$-DMSO): δ3.34 (s, 2H, ArCH2 $COO^-$), δ3.67(s, 3H, -OH), δ4.19(s, 6H, $-CH_2$-OH), δ6.53 (t, 1 H, *J* = 7.7Hz, N-Ar$^5$H), δ7.13(d, 1 H, *J* = 8.1 Hz, N-ArH), δ7.22 (d, 1 H, *J* =6.9Hz, N-ArH), δ7.43 (d, 2H, *J* = 8.7Hz, Br-ArH), δ7.76 (d, 2H, *J* = 8.7Hz, Br-ArH), δ7.13 (s, 3H, $NH_3^+$), δ7.84 (bro, 2H, Ar-$NH_2$). The analysis results of MS, UV, IR, and HPLC showed the product was bromfenac trometamol salt, and had a purity of 99.6%.

Example 3: In vitro transdermal absorption test of bromfenac organic salts

Preparation of solutions of bromfenac salts: bromfenac sodium (reference), bromfenac diethylamine salt of Example 1, and bromfenac trometamol salt of Example 2 were precisely weighed separately to obtain 0.25g samples, which were separately placed in 25ml volumetric flasks, and phosphate buffered solution (pH value was 7.0) was used to reach the metered volume to obtain stock solutions for standby use.

Chromatography conditions: detector: ultraviolet absorption detector (wavelength: 266 nm). Chromatography column: octadecyl silica column (4.0mm×150mm, 5μm). Mobile phase: precisely weighed ammonium acetate 3.85g, added with water to the metered volume of 1000mL. 600 mL of this solution was taken, and added with 250mL of methanol and 150mL of tetrahydrofuran. Flow rate: 1.5mL/min.

Plotting standard curves: bromfenac sodium, bromfenac diethylamine salt and bromfenac trometamol salt in suitable amounts were precisely weighed separately, dissolved in mobile phase, diluted separately to form test solutions with concentrations of 10, 30, 60, 120, 300, 700μg/ml, which were separately weighed precisely to take 20μl and injected into chromatography and separately assayed. The sample peak area (A) was used a ordinate and concentrations of bromfenac sodium, bromfenac diethylamine salt and bromfenac trometamol salt (C) were used as abscissa for linear regression to obtain respectively standard curve equations: for bromfenac sodium, A = 0.0224 C + 0.0584 (*r*= 0.9998), for bromfenac diethylamine salt, *A* = 0.0217 *C* - 0.0186 *(r* = 0.9997), for bromfenac trometamol salt, *A* = 0.0231C - 0.0436 (*r* = 0.9999). The results showed that the concentrations of bromfenac salts in range of 10-700 μg/ml were of good linear correlation with peak areas.

Recovery test: suitable amounts of the stock solutions were precisely weighed, diluted with physiological saline to separately obtain solutions of bromfenac sodium, bromfenac diethylamine salt and bromfenac trometamol salt, for each of the salts, solutions with low, middle and high concentrations (80.0μg/ml, 100.0μg/ml, and 120.0μg/ml) were provided, and 3 samples were prepared for each concentration, and measurement was performed respectively, recovery rates were separately calculated as: bromfenac sodium (99.5%, 100.1%, 100.2%), bromfenac diethylamine (99.6%, 99.9%, 100.3%), bromfenac trometamol salt (99.6%, 100.0%, 100.4%).

Transdermal absorption test: ex vivo skins of back and abdomen of 200-260g rats were taken and used as osmotic membranes, which were fixed with suture on diffusion cells (diffusion cells having a diameter of 1.25cm, and an effective area of 1.23 $cm^2$). Stock solutions of bromfenac sodium, bromfenac diethylamine salt and bromfenac trometamol salt in an amount of 2ml were separately added to supplying chamber, 100ml of physiological saline was used as receiving solution in receiving cells, kept at a constant temperature of 32°C, stirred (250r/min). 5ml samples of receiving solutions were taken precisely with time intervals of 0, 2, 4, 6, 8, 10, 12, 24hours, filtered, measured according to the above method to determine first-order light spectrum derivative absorption values and calculate contents, and accumulative osmolality Q was calculated according to the following formula;

$$Q = \frac{\sum_{i=1}^{n-1} c_i \times v_1 + c_n \times v_2}{A}$$

In the above formula, *c* is concentration of receiving solution sample; *v*1 is volume of sample as taken at every turn; *v*2 is volume of diffusion cell; A is diffusion area. Diagrams were plotted by using t as abscissa, Q as ordinate to obtain osmosis curves of bromfenac sodium, bromfenac diethylamine salt and bromfenac trometamol salt; linear regression was performed using accumulative osmolality *Q* against time, the obtained slope K was transdermal osmotic rate; the accumulative osmolality of 24hours was multiplied by the effective osmotic area (1.23 $cm^2$) to obtain total osmolality of 24hours; and the total osmolality of 24hours was divided by the mass of the added product in the supplying chamber to obtain osmotic percentage.

Results: accumulative osmolalities of bromfenac salts at different time were shown in Table 1, and osmotic rates, total osmolality of 24hours, and osmotic percentages were shown in Table 2.

Table 1: Accumulative osmolalities of three drugs at different time (μg/$cm^2$, n=3)

| Sample group | 2hours | 4hours | 6hours | 8hours | 10hours | 12hours | 24hours |
|---|---|---|---|---|---|---|---|
| Bromfenac sodium group_ (reference) | 45.5 | 118.5 | 340.8 | 708.6 | 1231.3 | 2214.5 | 4554.1 |
| Bromfenac diethylamine salt group | 322.8 | 964.1 | 1898.6 | 2721.8 | 3303.0 | 4123.6 | 8426.7 |
| Bromfenac trometamol salt group | 277.2 | 632.5 | 1268.3 | 2089.6 | 2736.1 | 3085.7 | 7326.9 |

Table 2: Transdermal absorption data of three drugs

| Sample group | Osmotic rate μg/cm$^2$.h$^{-1}$ | Total osmolality of 24h, μg | Osmotic percentage, % |
|---|---|---|---|
| Bromfenac sodium group_ (reference) | 219.05 | 5600.0 | 28.0 |
| Bromfenac diethylamine salt group | 369.04 | 10360.0 | 51.80 |
| Bromfenac trometamol salt group | 320.61 | 9010.0 | 45.05 |

Analysis of results:

It can be seen from Table 1 and Table 2 that Bromfenac diethylamine salt and bromfenac trometamol salt of the present invention as well as bromfenac sodium (reference) all have certain transdermal absorption effects, in which the total osmolality of 24hours of bromfenac diethylamine salt was the highest, while that of bromfenac trometamol salt was the second, but still higher than that of bromfenac sodium. The results showed that the in vitro transdermal absorption effects of bromfenac diethylamine salt and bromfenac trometamol salt were significantly better than those of bromfenac sodium, and their transdermal absorption amounts were 1.85 times and 1.61 times that of bromfenac sodium separately. This indicates that the two new bromfenac organic salts of the present invention have excellent transdermal absorption effects, and are promising in clinical application.

Example 4: Tissue distribution test of bromfenac organic salts

Preparation of bromfenac salt solutions: 0.25g samples of bromfenac sodium (reference), bromfenac diethylamine salt as prepared in Example 1 and bromfenac trometamol salt as prepared in Example 2 were precisely weighed separately, placed in 25ml volumetric flasks respectively, metered volume to obtain stock solutions for standby use.

Chromatography conditions: detector: ultraviolet absorption detector (wavelength: 266nm). Chromatography column: octadecyl silica gel column (4.6mm×150mm, 5μm). Mobile phase: precisely weighed ammonium acetate 3.85g, added with water to the metered volume of 1000mL. 700mL of this solution was taken and added with 300mL acetonitrile. Flow rate: 1.0mL/min.

Plotting standard curves: 200 μl samples of blank tissue were taken, separately added with 50μl of series standard solutions of bromfenac sodium, bromfenac diethylamine salt and bromfenac trometamol salt separately, added with 50μl of internal standard respectively, to form sample solutions with equivalent tissue concentrations of bromfenac sodium, bromfenac diethylamine salt and bromfenac trometamol salt as 50, 100, 250, 500, 1000, 5000, 10000ng/ml, which were separately weighed precisely to take 20μl and injected into chromatography and separately assayed. The concentration of substance to be tested (X) was abscissa, and the ratio of the peak area of the substance to be tested to that of the internal standard (Y) was ordinate, and linear regression calculation was conducted by weighted least square method (weight coefficient was $1/X^2$) to obtain a linear regression equation as standard curvilinear equation, that was, for bromfenac sodium, $Y= 0.000083X+ 0.01205$ ($r$ = 0.998), for bromfenac diethylamine salt, $Y = 0.000082X- 0.00013$ ($r$ = 0.9998), for bromfenac trometamol salt, $Y = 0.000096X - 0.00361$ ($r$ = 0.996). The results showed that the concentrations of bromfenac salts in range of 50-10000 ng/ml were of good linear correlation with peak areas.

Recovery test: 200μl of blank tissue samples were taken to separately prepare tissue samples of bromfenac sodium (reference), bromfenac diethylamine salt and bromfenac trometamol salt, and for each of the salts, solutions with low, middle and high concentrations (100.0ng/ml, 500.0ng/ml, and 5000ng/ml) were provided, and 3 samples were prepared for each concentration, and measurement was performed respectively, recovery rates were separately calculated as: bromfenac sodium (85.7%, 83.8%, 83.2%), bromfenac diethylamine (86.6%, 85.5%, 84.3%), Bromfenac trometamol salt (84.6%, 83.0%, 84.4%).

Tissue distribution measurement:

1) On the test day, removed short hair on the back of rats were with a electric hair clipper to expose surface layer of skin, while trying to avoid breaking skin during hair cutting, and then washed unhairing region with a large quantity of water;
2) anesthetized rats using 10% chloral hydrate in anesthetic agent dose of 3.5ml/kg;
3) randomly divided the rats into 3 groups, 15 rats per group. On the unhairing region of back, the rats of 3 groups were separately administered with 1g of bromfenac sodium, bromfenac diethylamine and Bromfenac trometamol preparations, and the administration regions were covered with freshness-keeping plastic film;

4) executed rats before administration (0 hours) and at 2hours, 4hours, 6hours, 8hours, 10hours after administration, took muscle tissues under skin of administration sites;
5) simply washed muscle tissues with physiological saline, then absorbed excessive water with filter paper;
6) precisely weighed 1g of muscle tissue, cut into pieces, homogenizing with 3ml of physiological saline in electric grinding mill. A homogeneous muscle tissue homogenate was obtained after muscle tissue was ground completely;
7) took 200μL of tissue sample, added with 50μL of an internal standard solution, mixed homogeneously, added with 1 mL of methanol, mixed by vortex for 1 min, centrifuged for 5 min (3500 rpm), separated and placed upper layer organic phase into another tube, blow-dried with 37°C air, dissolved the residue by adding 100 μL of mobile phase, mixed by vortex, and took 20 μL to perform HPLC analysis.

Results: Concentration distributions of bromfenac salts in muscle tissue at different time are shown in Table 3.

Table 3: Concentration distributions of three drugs in muscle tissue at different time (ng/ml, n=3)

| Sample group | 0hours | 2hours | 4hours | 6hours | 8hours | 10hours |
|---|---|---|---|---|---|---|
| Bromfenac sodium group (reference) | 0 | 238.5 | 693.1 | 508.6 | 312.8 | 130.5 |
| Bromfenac diethylamine salt group | 0 | 1092.1 | 2657.6 | 1803.7 | 1108.3 | 262.4 |
| Bromfenac trometamol salt group | 0 | 851.7 | 1492.3 | 1098.9 | 789.5 | 201.2 |

It can be seen from data of Table 3 that the bromfenac diethylamine salt and bromfenac trometamol salt of the present invention as well as bromfenac sodium (reference) all have good distribution in tissue, in which the distributions of bromfenac diethylamine salt, bromfenac trometamol salt and bromfenac sodium in tissue (muscle tissue, especially muscle tissue under skin) reach maximum values at 2hours, i.e., reach peak concentrations. The results show that the concentrations of bromfenac diethylamine salt and bromfenac trometamol salt as distributed in tissue are significantly greater than that of bromfenac sodium, which indicates that in comparison with bromfenac sodium, the two new bromfenac organic salts of the present invention can exhibit better local potency and have good clinical application value.

Example 5:Test of anti-inflammatory activity of bromfenac organic salts

In the present example, the anti-inflammatory activities of bromfenac sodium (reference), bromfenac diethylamine salt and bromfenac trometamol salt were measured by test of effects of bromfenac salts on swelling of auricle caused by xylene in mice and on swelling of foot caused by egg white in rats.

1. Test of effects on swelling of auricle caused by xylene in mice:

Test grouping: 40 mice, bodyweight 20±2 g, randomly divided into 4 groups, 10 mice per group, i.e., physiological control group, bromfenac sodium group, bromfenac diethylamine salt group, and bromfenac trometamol salt group.
Preparation of solutions of bromfenac salts: 1 g samples of bromfenac sodium, bromfenac diethylamine salt as prepared in Example 1 and bromfenac trometamol salt as prepared in Example 2 were seperately weighed precisely, placed in 100ml volumetric flasks respectively, added with distilled water to reach the metered volume to obtain stock solutions for standby use.
Test method: the mice of each test group were administered with physiological saline, solution of bromfenac sodium, solution of bromfenac diethylamine salt and solution of bromfenac trometamol salt, twice per day via lavage in lavage dose of 50mL/Kg, for consecutive 7 days, 1 h after the last administration, xylene in dose of 0.05mL/mouse was coated on right ears of mice of each test group, while their left ears were used as control, the mice were executed after 30 min, ear parts that were at the same place and had the same area were cut from both ears by a puncher with a diameter of 6 mm, and weighed with ten thousandth electronic analytical balance, and the weight difference between two ears was used as index of swelling. The results are shown in Table 4.

Table 4: Effects of Bromfenac sodium, Bromfenac diethylamine salt and Bromfenac trometamol salt on swelling of auricle in mice

| Group | Animal number (mice) | Swelling degree (mg) |
|---|---|---|
| Physiological saline control group | 10 | 11.31±1.13 |
| Bromfenac sodium group (reference) | 10 | 5.45±0.36 |

(continued)

| Group | Animal number (mice) | Swelling degree (mg) |
|---|---|---|
| Bromfenac diethylamine salt group | 10 | 5.37±0.50 |
| Bromfenac trometamol salt group | 10 | 5.41±0.47 |

As shown in Table 4, bromfenac sodium (reference), bromfenac diethylamine salt and bromfenac trometamol salt all have significant inhibition effects on swelling of auricle caused by xylene in mice, and the anti-inflammatory effects of bromfenac diethylamine salt and bromfenac trometamol salt are similar with that of bromfenac sodium.

2. Test of effects on swelling of foot caused by egg white in rats

Test grouping: 40 rats, bodyweight 200-220g, randomly divided into 4 groups, 10 rats per group, i.e., physiological saline control group, bromfenac sodium group, bromfenac diethylamine salt group, and bromfenac trometamol salt group.

Preparation of solutions of bromfenac salts: 1g samples of bromfenac sodium, bromfenac diethylamine salt as prepared in Example 1 and bromfenac trometamol salt as prepared in Example 2 were seperately weighed precisely, placed in 100ml volumetric flasks respectively, added with physiological saline to reach the metered volume to obtain stock solutions for standby use.

The rats of each test group were administered with physiological saline, solution of bromfenac sodium, solution of bromfenac diethylamine salt and solution of bromfenac trometamol salt, twice per day via lavage in lavage dose of 50mL/Kg, for consecutive 7 days, before the last administration, volumes of voix pedis of normal right and left hindfeet of each rat were measured, and the measurement was performed by blood capillary volume method. The linedrawing position at ankle joint of rat was adjusted so that the volumes of voix pedis of right and left hindfeet were about 1 mL, 0.5 hours after the last administration, 0.1mL of newly prepared 10% egg white liquid was subcutaneously injected to voix pedis of right and left hindfeet of rats respectively, and the volumes of voix pedis of right and left hindfeet of rats were measured again at 0.5, 1, 2, 4, 6 hours after injection of egg white, and swelling rate was calculated (voix pedis swelling rate = voix pedis volume at different time after injection of egg white - voix pedis volume at the same side before injection of egg white / voix pedis volume at the same side before injection of egg white), and the results were shown in Table 5.

Table 5: Test of effects on swelling of foot caused by egg white in rats

| Group | Animal number (rats) | voix pedis volume for different time of administration, mL(swelling rate) | | | | | |
|---|---|---|---|---|---|---|---|
| | | Normal rats | 0.5hours | 1.0hours | 2.0hours | 4.0hours | 6.0hours |
| Physiological control group | 10 | 1.00±0.08 (1.84±0.13) | 1.83±0.09 (0.84±0.13) | 1.73±0.08 (0.74±0.11) | 1.54±0.09 (0.55±0.11) | 1.35±009 (0.35±0.10) | 1.17±0.08 (0.17±0.08) |
| Bromfenac sodium group (reference) | 10 | 1.51±0.11 (0.58±0.06) | 1.56±0.11 (0.58±0.06) | 1.51±0.10 (0.53±0.07) | 1.39±0.11 (0.40±0.09) | 1.20±0.10 (0.22±0.08) | 1.04±0.06 (0.06±0.05) |
| Bromfenac diethylamine salt group | 10 | 1.61±0.08 (0.65±0.08) | 1.61±0.08 (0.65±0.08) | 1.55±0.09 (0.58±0.08) | 1.38±0.06 (0.41±0.08) | 1.21±0.06 (0.23±0.05) | 1.06±0.08 (0.08±0.06) |
| Bromfenac trometamol salt group | 10 | 1.59±0.09 (0.63±0.09) | 1.59±0.09 (0.63±0.09) | 1.54±0.11 (0.57±0.10) | 1.41±0.13 (0.44±0.13) | 1.24±0.10 (0.20±0.10) | 1.08±0.08 (0.10±0.07) |

As shown in Table 5, in comparison with physiological saline control group, bromfenac sodium (reference), bromfenac diethylamine salt and bromfenac trometamol salt could significantly inhibit voix pedis swelling caused by egg white in rats within 0.5hours to 6hours, so that swelling rates decreased, and the three salts had similar inhibition level on swelling.

According to the results of the test of effects on swelling of auricle caused by xylene in mice and the test of effects on swelling of foot caused by egg white in rats, it can be seen that bromfenac diethylamine salt and bromfenac trometamol salt as well as bromfenac sodium all can significantly inhibit swelling, and have similar inhibition level, which indicates that bromfenac diethylamine salt and bromfenac trometamol salt as well as bromfenac sodium have similar anti-inflammatory activity.

Example 6: Test of stability of Bromfenac organic salts

1) Chemical stability of bromfenac organic salt in solid state

Bromfenac sodium (reference), bromfenac diethylamine salt as prepared in Example 1 and bromfenac trometamol salt as prepared in Example 2 were subjected to accelerated stability test at 60°C, the results were shown in Table 6.

HPLC analysis conditions:

Detector: ultraviolet absorption detector (wavelength: 266nm).
Chromatography column: octadecyl silica gel column (4.0mm×150mm, 5um).
Mobile phase: 3.85g of ammonium acetate was precisely weighed, added with water to reach the metered volume of 1000mL. 600mL of this solution was taken and added with 250mL of methanol and 150mL of tetrahydrofuran.
Flow rate: 1.5mL/min.

Table 6: Chemical stability of bromfenac organic salts in solid state

| Sample name | Storage cycle | | | |
|---|---|---|---|---|
| | Initial | 1 week | 2 weeks | 3 weeks |
| Bromfenac sodium (reference) | 99.6% | 99.5% | 99.3% | 99.1% |
| Bromfenac diethylamine salt | 99.5% | 99.5% | 99.3% | 99.2% |
| Bromfenac trometamol salt | 99%.6 | 99. %6 | 99.4% | 99.2% |

As shown in Table 6, in the accelerated test at 60°C, the contents of bromfenac sodium (reference), bromfenac diethylamine salt as prepared in Example 1 and bromfenac trometamol salt as prepared in Example 2 had no significant substantial change, which indicated when bromfenac diethylamine salt and bromfenac trometamol salt are compared with bromfenac sodium, bromfenac diethylamine salt and bromfenac trometamol salt also have excellent chemical stability.

2) Chemical stability of bromfenac organic salts in aqueous state

In order to measuring stability in aqueous state, the aqueous solutions of bromfenac sodium, bromfenac diethylamine salt and bromfenac trometamol salt of Example 3 were stored in completely dark place at 25°C for 4 weeks, then the contents of Bromfenac organic salts were measured by HPLC method under the same conditions for stability test in solid state, and the results were shown in Table 7.

Table 7: Chemical stability of bromfenac organic salts in aqueous state

| Sample name | Storage cycle | | | |
|---|---|---|---|---|
| | Initial | 1 week | 2 weeks | 3 weeks |
| Bromfenac sodium (reference) | 99.6% | 99.5% | 99.4% | 99.1% |
| Bromfenac diethylamine salt | 99.5% | 99.5% | 99.4% | 99.2% |

(continued)

| Sample name | Storage cycle | | | |
|---|---|---|---|---|
| | Initial | 1 week | 2 weeks | 3 weeks |
| Bromfenac trometamol salt | 99%.6 | 99. %6 | 99.3% | 99.1% |

The results of stability test under dark conditions showed that degradation products and content changes were not observed in the aqueous solutions of bromfenac sodium (reference), bromfenac diethylamine salt and bromfenac trometamol salt.

In sum, bromfenac diethylamine salt and bromfenac trometamol salt are prone to crystallization, have no moisture absorption, have stable chemical properties, have good water solubility, and have good lipo solubility as well, so that transdermal and transmembrane absorbance are enhanced; they have equivalent anti-inflammatory and analgesic activity in comparison with bromfenac sodium, and have elevated concentration as distributed in tissue (muscle tissue, especially muscle tissue under skin). Hence, they can be used as non-steroid anti-inflammatory agents for in vitro topical administration.

The scope of the present invention is given by appended claims.

## Claims

1. A bromfenac organic salt, which is bromfenac diethylamine salt having the structure of Formula II, or bromfenac trometamol salt having the structure of Formula III:

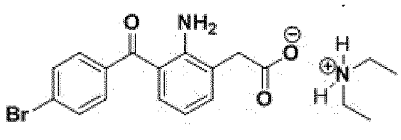

II

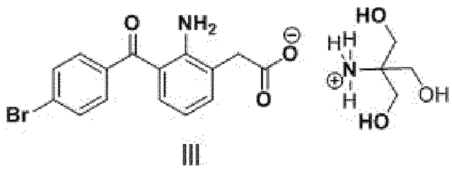

III

2. A method for preparing the bromfenac organic salt according to claim 1, comprising the following steps:

   1) dissolving bromfenac in an organic solvent, heating to dissolve, then adding an organic base in equimolar amount, stirring until the end of reaction;
   2) concentrating and evaporating the reaction solution to dry to obtain a light yellow or orange yellow solid;
   3) recrystallizing the solid obtained in step 2) to obtain the bromfenac organic salt; wherein the said organic base is diethylamine or trometamol.

3. The method according to claim 2, wherein said organic solvent in step 1) is one or more selected from the group consisting of methanol, ethanol, acetone, n-butanol and toluene.

4. The method according to claim 3, wherein said organic solvent in step 1) is acetone.

5. The method according to any one of the claims 2-3, wherein the temperature of heating in step 1) is 25-80°C.

6. The method according to claim 5, wherein the temperature of heating in step 1) is 45-65°C.

7. The method according to claim 6, wherein the temperature of heating in step 1) is 55°C.

8. The method according to any one of the claims 2-7, wherein the time for stirring and reacting in step 1) is 0.5-2 hours.

9. The method according to claim 8, wherein the time for stirring and reacting in step 1) is 1 hour.

10. The method according to any one of the claims 2-9, wherein the recrystallization in step 3) is performed at 0°C or below.

11. The method according to claim 10, wherein the recrystallization in step 3) is performed at -5°C.

**12.** The method according to any one of the claims 2-11, further comprising step 4): vacuum drying the crystal obtained by recrystallizing.

**13.** A pharmaceutical composition comprising the bromfenac organic salt according to claim 1, wherein the composition is in a dosage form selected from a tablet, a capsule, a dripping pill, an aerosol, a pills, a powder, an emulsion, a granule, a liposome, a transdermal agent, a buccal tablet, a suppository, and a freeze-dried powder injection.

**14.** The bromfenac organic salt of claim 1 or the pharmaceutical composition of claim 13 for use as a medicament.

**15.** The bromfenac organic salt according to claim 1 or the pharmaceutical composition according to claim 13, for use in the treatment and/or prophylaxis of an inflammation or for analgesia.

**16.** The bromfenac organic salt or the pharmaceutical composition for use according to claim 15, wherein the inflammation is an inflammation associated with or mediated by prostaglandin.

**17.** The bromfenac organic salt or the pharmaceutical composition for use according to claim 15, wherein the inflammation is a skin inflammation or ear inflammation or eye inflammation.

**18.** The bromfenac organic salt or the pharmaceutical composition for use according to claim 15, wherein the inflammation is blepharitis, conjunctivitis, keratohelcosis, or ocular postoperative inflammation.

**19.** The bromfenac organic salt or the pharmaceutical composition for use according to claim 15, wherein the analgesia is to relieve a pain caused or mediated by prostaglandin.

**20.** The bromfenac organic salt or the pharmaceutical composition for use according to claim 15, wherein the analgesia is to relieve a pain caused by an ocular disease.

**Patentansprüche**

**1.** Organisches Bromfenac-Salz, das Bromfenac-Diethylaminsalz ist, welches die Struktur der Formel II aufweist oder Bromfenac-Trometamolsalz, welches die Struktur der Formel III aufweist:

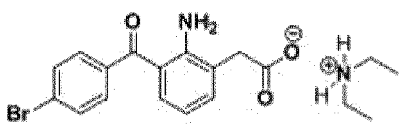

II

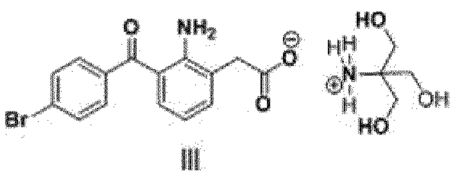

III

**2.** Verfahren zum Herstellen des organischen Bromfenac-Salzes nach Anspruch 1, das die folgenden Schritte umfasst:

1) Lösen von Bromfenac in einem organischen Lösemittel, Erhitzen bis zum Auslösen, daraufhin Zugeben einer organischen Base in äquimolarer Menge, Rühren bis zum Ende der Reaktion;
2) Konzentrieren und Eindampfen der Reaktionslösung zum Trocknen, um einen hellgelben oder orangegelben Feststoff zu erhalten;
3) Umkristallisieren des in Schritt 2) erhaltenen Feststoffs, um das organische Bromfenac-Salz zu erhalten; wobei die organische Base Diethylamin oder Trometamol ist.

**3.** Verfahren nach Anspruch 2, wobei das organische Lösemittel in Schritt 1) eines oder mehrere ausgewählt aus der Gruppe bestehend aus Methanol, Ethanol, Aceton, n-Butanol und Toluol ist.

**4.** Verfahren nach Anspruch 3, wobei das organische Lösemittel in Schritt 1) Aceton ist.

**5.** Verfahren nach einem der Ansprüche 2-3, wobei die Temperatur des Erhitzens in Schritt 1) 25-80 °C beträgt.

**6.** Verfahren nach Anspruch 5, wobei die Temperatur des Erhitzens in Schritt 1) 45-65 °C beträgt.

7. Verfahren nach Anspruch 6, wobei die Temperatur des Erhitzens in Schritt 1) 55 °C beträgt.

8. Verfahren nach einem der Ansprüche 2-7, wobei die Zeit für das Rühren und Umsetzen in Schritt 1) 0,5-2 Stunden beträgt.

9. Verfahren nach Anspruch 8, wobei die Zeit für das Rühren und Umsetzen in Schritt 1) 1 Stunde beträgt.

10. Verfahren nach einem der Ansprüche 2-9, wobei die Umkristallisation in Schritt 3) bei 0 °C oder darunter durchgeführt wird.

11. Verfahren nach Anspruch 10, wobei die Umkristallisation in Schritt 3) bei -5 °C durchgeführt wird.

12. Verfahren nach einem der Ansprüche 2-11, das ferner Schritt 4) umfasst: Vakuumtrocknen des durch Umkristallisieren erhaltenen Kristalls.

13. Pharmazeutische Zusammensetzung, die das organische Bromfenac-Salz nach Anspruch 1 umfasst, wobei die Zusammensetzung in einer Dosierungsform vorliegt, die ausgewählt ist aus einer Tablette, einer Kapsel, einer Tropfenpille, einem Aerosol, einer Pille, einem Pulver, einer Emulsion, einem Granulat, einem Liposom, einem transdermalen Mittel, einer bukkalen Tablette, einem Suppositorium und einer gefriergetrockneten Pulverinjektion.

14. Organisches Bromfenac-Salz nach Anspruch 1 oder pharmazeutische Zusammensetzung nach Anspruch 13 zur Verwendung als Arzneimittel.

15. Organisches Bromfenac-Salz nach Anspruch 1 oder pharmazeutische Zusammensetzung nach Anspruch 13 zur Verwendung bei der Behandlung und/oder Prophylaxe einer Entzündung oder zur Analgesie.

16. Organisches Bromfenac-Salz oder pharmazeutische Zusammensetzung zur Verwendung nach Anspruch 15, wobei die Entzündung eine Entzündung ist, die mit Prostaglandin verbunden ist oder dadurch vermittelt wird.

17. Organisches Bromfenac-Salz oder pharmazeutische Zusammensetzung zur Verwendung nach Anspruch 15, wobei die Entzündung eine Hautentzündung oder eine Ohrenentzündung oder eine Augenentzündung ist.

18. Organisches Bromfenac-Salz oder pharmazeutische Zusammensetzung zur Verwendung nach Anspruch 15, wobei die Entzündung Blepharitis, Konjunctivitis, Keratohelkose oder eine postoperative Okularentzündung ist.

19. Organisches Bromfenac-Salz oder pharmazeutische Zusammensetzung zur Verwendung nach Anspruch 15, wobei die Analgesie von einem durch Prostaglandin verursachten oder vermittelten Schmerz befreien soll.

20. Organisches Bromfenac-Salz oder pharmazeutische Zusammensetzung zur Verwendung nach Anspruch 15, wobei die Analgesie von einem durch eine Okularerkrankung verursachten Schmerz befreien soll.

## Revendications

1. Sel organique de bromfénac, qui est un sel de diéthylamine de bromfénac ayant la structure de formule II, ou un sel de trométamol de bromfénac ayant la structure de formule III :

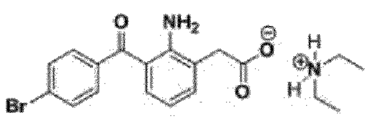

II

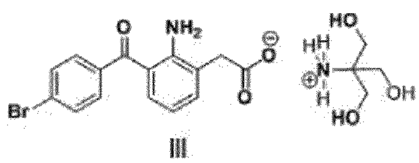

III

.

2. Procédé de préparation du sel organique de bromfénac selon la revendication 1, comprenant les étapes consistant à :

1) dissoudre le bromfénac dans un solvant organique, le chauffer pour le dissoudre, puis ajouter une base organique en une quantité équimolaire, agiter jusqu'à la fin de la réaction ;

2) concentrer et évaporer la solution réactionnelle jusqu'à siccité pour obtenir un solide jaune clair ou jaune orangé ;
3) recristalliser le solide obtenu dans l'étape 2) pour obtenir le sel organique de bromfénac ;

dans lequel ladite base organique est la diéthylamine ou le trométamol.

**3.** Procédé selon la revendication 2, dans lequel ledit solvant organique de l'étape 1) est un ou plusieurs solvants sélectionnés dans le groupe constitué par le méthanol, l'éthanol, l'acétone, le n-butanol et le toluène.

**4.** Procédé selon la revendication 3, dans lequel ledit solvant organique de l'étape 1) est l'acétone.

**5.** Procédé selon l'une quelconque des revendications 2 à 3, dans lequel la température de chauffage de l'étape 1) est de 25 à 80 °C.

**6.** Procédé selon la revendication 5, dans lequel la température de chauffage de l'étape 1) est de 45 à 65 °C.

**7.** Procédé selon la revendication 6, dans lequel la température de chauffage de l'étape 1) est 55 °C.

**8.** Procédé selon l'une quelconque des revendications 2 à 7, dans lequel le temps d'agitation et de réaction dans l'étape 1) est de 0,5 à 2 heures.

**9.** Procédé selon la revendication 8, dans lequel le temps d'agitation et de réaction dans l'étape 1) est de 1 heure.

**10.** Procédé selon l'une quelconque des revendications 2 à 9, dans lequel la recristallisation dans l'étape 3) est réalisée à 0 °C ou au-dessous.

**11.** Procédé selon la revendication 10, dans lequel la recristallisation dans l'étape 3) est réalisée à -5 °C.

**12.** Procédé selon l'une quelconque des revendications 2 à 11, comprenant en outre l'étape 4) consistant à : sécher sous vide le cristal obtenu par recristallisation.

**13.** Composition pharmaceutique comprenant le sel organique de bromfénac selon la revendication 1, dans laquelle la composition est sous une forme posologique sélectionnée parmi un comprimé, une gélule, un minicomprimé sphérique obtenu par stillation (dripping pill), un aérosol, une pilule, une poudre, une émulsion, un granule, un liposome, un agent transdermique, un comprimé buccal, un suppositoire et une injection de poudre lyophilisée.

**14.** Sel organique de bromfénac selon la revendication 1 ou composition pharmaceutique selon la revendication 13, pour une utilisation en tant que médicament.

**15.** Sel organique de bromfénac selon la revendication 1 ou composition pharmaceutique selon la revendication 13, pour une utilisation dans le traitement et/ou la prophylaxie d'une inflammation ou pour une analgésie.

**16.** Sel organique de bromfénac ou composition pharmaceutique pour une utilisation selon la revendication 15, dans lequel/laquelle l'inflammation est une inflammation associée à ou médiée par la prostaglandine.

**17.** Sel organique de bromfénac ou composition pharmaceutique pour une utilisation selon la revendication 15, dans lequel/laquelle l'inflammation est une inflammation cutanée ou une inflammation auriculaire ou une inflammation oculaire.

**18.** Sel organique de bromfénac ou composition pharmaceutique pour une utilisation selon la revendication 15, dans lequel/laquelle l'inflammation est une blépharite, une conjonctivite, une kératolyse ou une inflammation postopératoire oculaire.

**19.** Sel organique de bromfénac ou composition pharmaceutique pour une utilisation selon la revendication 15, dans lequel/laquelle l'analgésie est destinée à soulager une douleur provoquée ou médiée par la prostaglandine.

**20.** Sel organique de bromfénac ou composition pharmaceutique pour une utilisation selon la revendication 15, dans lequel/laquelle l'analgésie est destinée à soulager une douleur provoquée par une maladie oculaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2007021507 A1
- US 2007082857 A1
- US 2003055051 A1